(19) Europäisches Patentamt
European Patent Office
Office européen des brevets

(11) EP 2 829 264 B1

(12) EUROPEAN PATENT SPECIFICATION

(45) Date of publication and mention of the grant of the patent:
**14.02.2018 Bulletin 2018/07**

(21) Application number: **13764778.0**

(22) Date of filing: **19.03.2013**

(51) Int Cl.:
***A61K 8/63*** *(2006.01)* ***A61K 8/37*** *(2006.01)*
***A61K 8/39*** *(2006.01)* ***A61K 8/44*** *(2006.01)*
***A61K 8/55*** *(2006.01)* ***A61K 8/67*** *(2006.01)*
***A61Q 19/00*** *(2006.01)*

(86) International application number:
**PCT/JP2013/057901**

(87) International publication number:
**WO 2013/141263 (26.09.2013 Gazette 2013/39)**

(54) **HIGHLY TRANSPARENT EMULSION COMPOSITION AND HIGHLY TRANSPARENT COSMETIC**

HOCHTRANSPARENTE EMULSIONSZUSAMMENSETZUNG UND HOCHTRANSPARENTES KOSMETIKUM DAMIT

COMPOSITION D'ÉMULSION HAUTEMENT TRANSPARENTE ET COSMÉTIQUE HAUTEMENT TRANSPARENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.03.2012 JP 2012066379**

(43) Date of publication of application:
**28.01.2015 Bulletin 2015/05**

(73) Proprietor: **FUJIFILM Corporation**
**Tokyo 106-8620 (JP)**

(72) Inventors:
- **IMAIZUMI, Yuuki**
**Ashigarakami-gun**
**Kanagawa 258-8577 (JP)**
- **AIMI, Makiko**
**Ashigarakami-gun**
**Kanagawa 258-8577 (JP)**

(74) Representative: **Klunker IP Patentanwälte PartG mbB**
**Destouchesstraße 68**
**80796 München (DE)**

(56) References cited:
**WO-A1-2004/096169**
**JP-A- H09 176 035**
**JP-A- 2000 247 866**
**JP-A- 2001 354 518**
**JP-A- 2003 104 886**
**JP-A- 2003 300 853**
**JP-A- 2003 321 347**
**JP-A- 2003 335 627**
**JP-A- 2005 179 241**
**JP-A- 2005 220 084**
**JP-A- 2005 350 378**
**JP-A- 2009 149 692**
**US-A1- 2005 143 324**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 829 264 B1

## Description

### Technical Field

This invention relates to a highly transparent emulsion composition and a highly transparent cosmetic.

### Background Art

Various types of oil-soluble medicinal agents are blended into cosmetics and the like. In general, oil-soluble medicinal agents are used in milky lotions, creams, and the like. Examples of the oil-soluble medicinal agents include glycyrrhetic acid derivatives such as stearyl glycyrrhetinate. Such an oil-soluble medicinal agent is blended into cosmetics and the like as an agent having an anti-inflammatory effect.

Meanwhile, in a case in which an oil-soluble medicinal agent such as a glycyrrhetic acid derivative is blended into an aqueous composition such as a lotion, it is necessary to solubilize the blend oil-soluble medicinal agent with a surfactant for blending it into the aqueous composition, or blend the oil-soluble medicinal agent into an oil-phase component of an emulsion composition using a large amount of oil agent or emulsifier and then blend the resultant emulsion composition with the aqueous composition.

As a technique relating to an emulsion composition prepared using a surfactant, an emulsion dispersion including an alkylated polysaccharide in combination with a phospholipid and an ionic surfactant is disclosed, for example, in Japanese Patent No. 4524098.

Japanese Patent No. 4341983 discloses a fine dispersion composition of an oil-soluble medicinal agent containing wax.

As a technique of easily emulsifying or solubilizing a poor water-soluble physiologically active substance in water by using a phospholipid, Japanese Patent Application Laid-Open (JP-A) No. 2007-197328 discloses a complex of a poor water-soluble physiologically active substance and phospholipid obtained by removing an organic solvent from a solution in which the poor water-soluble physiologically active substance and phospholipid are homogenously dissolved in the organic solvent to precipitate the poor water-soluble physiologically active substance and phospholipid simultaneously.

## SUMMARY OF INVENTION

### Technical Problem

However, since the ionic surfactant is used in the technique relating to an emulsion composition described in Japanese Patent No. 4524098, the emulsion composition is predicted to have skin irritancy.

The dispersion composition disclosed in Japanese Patent No. 4341983 is a dispersion composition of a solid or semisolid wax. Therefore, the dispersion composition may have a low transparent appearance, and in a case in which the solubility of the oil-soluble medicinal agent is low, the dispersion composition may contain only a small amount of a medicinal agent.

The technique described in JP-ANo. 2007-197328 employs an organic solvent and requires a spray drying process, and it is predicted that the scale of a manufacturing facility will be enlarged and cost will be increased. Furthermore, consideration of the safety of residual solvents with respect to organisms is sometimes required.

In a case in which a surfactant is used to solubilize an oil-soluble medicinal agent such as a glycyrrhetic acid derivative, it is predicted that the kind and the blending amount of the surfactant are restricted in consideration of skin irritancy. Associated with this, it is predicted that the kind and the blending amounts of an oil, a fragrance, and a medicinal agent including an oil-soluble medicinal agent to be blended are also restricted.

Meanwhile, in a case in which the emulsion composition containing an oil-soluble medicinal agent is applied to cosmetics, rich feeling upon use is provided due to an oil agent blended therein. However, in some cases, such cosmetics have inferior feeling upon use due to increased stickiness caused by an oil agent or an emulsifier.

Furthermore, transparency is often required for the emulsion composition to be applied to cosmetic formulations. Meanwhile, light entering a cosmetic formulation containing an emulsion composition is scattered depending on respective elements including the particle diameter of the emulsion composition, the refractive index of an oil agent, and the content of the oil agent. In a case in which each of these elements has a large value, the emulsion composition may become clouded, which may result in deterioration of transparency and provide an undesirable appearance.

As an approach for reducing turbidity of a cosmetic formulation containing an emulsion composition, the particle diameter of the emulsion composition is reduced for example. However, in order to reduce the particle diameter, it is necessary to increase the amount of the surfactant, which is undesirable in terms of skin irritancy and feeling upon use, particularly in a case in which the emulsion composition is applied to cosmetics. As an alternative approach, the particle

diameter is reduced by lowering the concentration of an oil-soluble medicinal agent contained in the emulsion composition to reduce the amount of an oil agent. However, it is necessary to add a large amount of emulsion composition in order to apply it to cosmetic formulations. This results in an increase in the content of the oil agent and the surfactant, and such a cosmetic may have skin irritancy and deteriorated feeling upon use.

Therefore, an emulsion composition that contains an oil-soluble medicinal agent such as a glycyrrhetic acid derivative in high concentration and exhibits excellent transparency and stability over time and a cosmetic that contains an emulsion composition, exhibits excellent transparency and stability over time, and has excellent feeling upon use are desired. However, such an emulsion composition and cosmetic have yet to be provided under the current conditions.

The invention has been made in view of the above circumstances and is intended to provide an emulsion composition that contains a glycyrrhetic acid derivative in high concentration and exhibits excellent transparency and stability over time.

Furthermore, the invention is intended to provide a cosmetic that contains a glycyrrhetic acid derivative in high concentration, exhibits excellent transparency and stability over time, and has excellent feeling upon use.

Solution to Problem

The present invention is defined in the appended claims. Means for solving the above problems are as follows.

[1] An emulsion composition comprising the following components (A) to (E):

(A) a glycyrrhetic acid derivative represented by the following Formula (1);
(B) at least one oil agent selected from the group consisting of tocopherol, tocopherol acetate and tocotrienol;
(C) a phospholipid;
(D) a water-soluble surfactant; and
(E) a fatty acid glyceride,

wherein a content of the glycyrrhetic acid derivative represented by Formula (1) is from 1.0% by mass to 5.0% by mass:

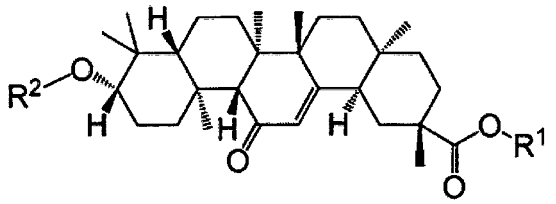

(1)

wherein, in Formula (1), with regard to $R^1$ and $R^2$, $R^1$ represents $-C_{12}H_{25}$, $-C_{14}H_{29}$, $-C_{16}H_{33}$, $-C_{18}H_{37}$, or $-C_{20}H_{41}$, and $R^2$ represents a hydrogen atom; or $R^1$ represents a hydrogen atom, and $R^2$ represents $-(C{=}O)C_{11}H_{23}$, $-(C{=}O)C_{13}H_{27}$, $-(C{=}O)C_{15}H_{31}$, or $-(C{=}O)C_{17}H_{35}$, and
wherein a total content of component (B) and component (E) is from 10% by mass to 25% by mass.

[2] The emulsion composition according to [1], wherein component (A) comprises stearyl glycyrrhetinate.

[3] The emulsion composition according to [1] or [2], wherein component (D) comprises at least one selected from the group consisting of a sucrose fatty acid ester and a polyglyceryl fatty acid ester.

[4] The emulsion composition according to any one of [1] to [3], wherein component (C) comprises a lecithin.

[5] The emulsion composition according to any one of [1] to [4], wherein a content ratio of component (C) to a total content of component (B) and component (E) [C/(B+E)] is from 0.01 to 0.5 in terms of mass.

[6] A cosmetic comprising the emulsion composition according to any one of [1] to [5].

[7] The cosmetic according to [6], wherein an absorbance measured with light having a wavelength of 650 nm is from 0.001 to 0.2.

Advantageous Effects of Invention

According to the invention, an emulsion composition that contains a glycyrrhetic acid derivative in high concentration and exhibits excellent transparency and stability over time can be provided.

Furthermore, according to the invention, a cosmetic that contains a glycyrrhetic acid derivative in high concentration, exhibits excellent transparency and stability over time, and excellent feeling upon use can be provided.

DESCRIPTION OF EMBODIMENTS

Hereinbelow, the invention is explained in details.

In this specification, any numerical range expressed herein using "to" refers to a range including the numerical values before and after "to" as the minimum and maximum values, respectively.

In the present invention, the term "aqueous phase" is used as a term contrasting with "oil phase", regardless of the type of solvent.

In the present specification, the term "process" encompasses an independent process, as well as a process that cannot be clearly distinguished from another process but yet achieves the expected effect of the process of interest.

In a case in which the amount of a component in the composition is indicated in the invention, when there are plural substances corresponding to the component in the composition, the indicated amount means the total amount of the plural substances present in the composition, unless specifically stated otherwise.

*Emulsion Composition*

An emulsion composition according to the invention includes the following components (A) to (D):

(A) a glycyrrhetic acid derivative represented by the following Formula (1) (hereinafter, sometimes referred to as "glycyrrhetic acid derivative");
(B) at least one oil agent selected from the group consisting of tocopherol, tocopherol acetate and tocotrienol (hereinafter, sometimes referred to as "specific oil agent");
(C) a phospholipid; and
(D) a water-soluble surfactant, and
(E) a fatty acid glyceride,

in which the content of the glycyrrhetic acid derivative represented by Formula (1) is from 1.0% by mass to 5% by mass and wherein a total content of component (B) and component (E) is from 10% by mass to 25% by mass.

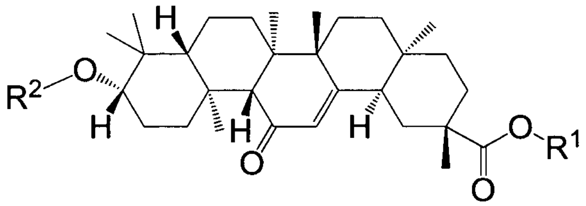

(1)

In Formula (1), with regard to $R^1$ and $R^2$, $R^1$ represents $-C_{12}H_{25}$, $-C_{14}H_{29}$, $-C_{16}H_{33}$, $-C_{18}H_{37}$, or $-C_{20}H_{41}$ and $R^2$ represents a hydrogen atom; or $R^1$ represents a hydrogen atom and $R^2$ represents $-(C{=}O)C_{11}H_{23}$, $-(C{=}O)C_{13}H_{27}$, $-(C{=}O)C_{15}H_{31}$, or $4C{=}O)C_{17}H_{35}$.

The emulsion composition according to the invention having such a configuration exhibits excellent transparency and stability over time, while containing the glycyrrhetic acid derivative in a concentration as high as 5% by mass to 10% by mass. Therefore, the effect expected from the glycyrrhetic acid derivative can be maintained over an extended time period.

The emulsion composition according to the invention is preferably an oil-in-water emulsion composition.

Here, the glycyrrhetic acid derivative is an oil-soluble substance and included in an emulsion composition as one of the oil phase components. Accordingly, in order to impart transparency to the emulsion composition containing the glycyrrhetic acid derivative, it is preferable to select an oil agent capable of dissolving the glycyrrhetic acid derivative without impairing the transparency of the emulsion composition.

The inventors found that transparency and stability over time, which becomes problematic in a case in which a high concentration of glycyrrhetic acid derivative is contained in the emulsion composition, can be achieved by selecting a specific oil agent in terms of the ability to dissolve the glycyrrhetic acid derivative and the refractive index thereof. The

inventors further found that feeling upon use (rich but non-sticky feeling), which is difficult to obtain in a case in which the emulsion composition according to the invention is applied to cosmetics, can be improved by reducing the total amount of the oil agent contained in the cosmetic and achieved the invention.

Hereinbelow, transparency as a criteria for selecting the oil agent is described.

• *Turbidity*

In the invention, turbidity is used as an index of transparency.

The turbidity in the invention is defined as an absorbance of a solution diluted with water in a predetermined dilution ratio measured with light having a wavelength of 650 nm at 25°C. The turbidity (absorbance) of the highly transparent emulsion composition according to the invention is preferably from 0.001 to 0.2, more preferably from 0.005 to 0.175, and still more preferably from 0.005 to 0.15.

In the invention, the absorbance (turbidity) as an index of "transparency" is measured with a spectrometer (V-630, manufactured by JASCO Corporation.).

In a case in which the particle diameter d of the emulsion composition is smaller than wavelength λ of visible light, the relationship between them and turbidity τ of the emulsion composition is represented by the following Equation A.

Formula A

$$\tau \propto n \frac{d^6}{\lambda^4}\left(\frac{m^2-1}{m^2+2}\right)^2$$

τ :turbidity、n:particle number、λ :wavelength
d:particle diameter、m:relative refractive index

As shown in Equation A, the particle diameter d of the emulsion composition, the particle number n of the emulsion composition, and the relative refractive index m of the oil agent contribute to the turbidity τ. Here, the particle number n of the emulsion composition is determined by the amount of the oil agent in the emulsion composition. In the case of the cosmetic containing the emulsion composition according to the invention, the turbidity thereof is determined by the amount of the emulsion composition to be blended into the cosmetic.

Here, the particle diameter and the particle number of the emulsion composition refer to the particle diameter and the particle number of the dispersed particles in the emulsion composition.

In order to improve the transparency of the emulsion composition, it is necessary to reduce turbidity. For this purpose, it is necessary to a) reduce the particle diameter of the emulsion composition, b) reduce the particle number of the emulsion composition, and c) reduce the relative reflective index of the oil agent.

Hereinbelow, the particle diameter and the particle number of the emulsion composition and the relative reflective index of the oil agent as factors for reducing turbidity are further explained.

• *Particle Diameter of Emulsion Composition*

As indicted in Equation A, turbidity depends on the sixth power of the particle diameter of the emulsion composition. Therefore, it is preferable to reduce the particle diameter of the emulsion composition in order to ensure transparency.

In general, the appearance of an emulsion composition is altered as follows in accordance with the change in the particle diameter of the emulsion composition.

That is, in a case in which the particle diameter of the emulsion composition is from 1 μm to 10μm, the emulsion composition has a white appearance since the light is scattered. The emulsion composition is tinged with blue and has a semitransparent appearance when the particle diameter thereof is decreased, and has a transparent appearance when the particle diameter thereof is further decreased.

The average particle diameter of the emulsion composition is preferably from 50 nm to 300 nm. In terms of transparency, the average particle diameter of the emulsion composition is preferably from 50 nm to 200 nm, and more preferably from 50 nm to 150 nm.

In order to achieve transparency with turbidity of from 0.001 to 0.2 in the emulsion composition according to the invention, the average particle diameter of the emulsion composition is preferably 200 nm or less, more preferably 150 nm or less, and still more preferably 100 nm or less.

The particle diameter of the dispersed particles contained in the emulsion composition can be measured with a commercially available particle size distribution analyzer or the like. Known examples of the measurement methods

for particle size distribution include optical microscopy, confocal laser microscopy, electron microscopy, atomic force microscopy, static light scattering, laser diffraction, dynamic light scattering, centrifugal sedimentation, electric pulse measurement, chromatography, and ultrasonic attenuation, and apparatuses corresponding to the respective principles are commercially available.

In terms of the particle diameter range and the ease of measurement, a dynamic light scattering method is used to measure the particle diameter of the dispersed particles in the invention. Examples of commercially available measuring apparatuses using dynamic light scattering include NANOTRACK UPA (Nikkiso Co., Ltd.), a dynamic light scattering type particle size distribution analyzer LB-550 (Horiba, Ltd.), and a concentrated system particle size analyzer FPAR-1000 (Otsuka Electronics Co., Ltd.).

As the average particle diameter in the invention, the value measured with a particle size analyzer FPAR-1000 (Otsuka Electronics Co., Ltd.) at 25°C is used.

• *Particle Number of Emulsion Composition*

Here, the particle number of the emulsion composition refers to the number of dispersed particles contained in the emulsion composition. Based on an assumption that the particle diameter is constant, the particle number is determined by the amount of the oil dispersed in the emulsion composition. In the case of the cosmetic, the particle number is determined by the amount of the emulsion composition to be blended. As the number of the dispersed particles contained in the emulsion composition increases, more light is scattered by the particles and turbidity is increased.

Here, the amount of the oil means the total amount of the oil agent contained in the emulsion composition.

It is not preferable to increase the amount of the oil in the emulsion composition since this leads to an increase in turbidity. In order to suppress the increase in turbidity, it is preferable to reduce the amount of the oil in the emulsion composition.

Furthermore, it is preferable to reduce the amount of the oil in the emulsion composition since an increase in the amount of the oil in the emulsion composition leads to not only an increase in turbidity but also deterioration of stability over time, and leads to deterioration of feeling upon use in a case in which the emulsion composition is applied to the cosmetic.

• *Relative Refractive Index of OilAgent*

The refractive index is an index representing how light travels in a substance.

As described above, the relative refractive index of the oil agent (i.e., the dispersed oil droplet) in the emulsion composition contributes to the turbidity of the emulsion composition. In the following description, the refractive index of the oil agent means the relative refractive index with respect to water at 20°C.

Turbidity increases as the refractive index of the emulsion composition increases and the difference between the refractive index of the emulsion composition and that of water (1.333 at 20°C) increases. Therefore, it is necessary to reduce the refractive index in order to reduce turbidity, and selection of the oil agent becomes important.

In general, it is known that the square of the refractive index is equal to the dielectric constant, and the refractive index tends to increase as the dielectric constant and the polarity of the oil agent increase.

As the refractive index in the invention, the value measured with a refractive index measuring device (RX-5000$\alpha$, manufactured by Atago Co. Ltd.) is used.

Hereinbelow, essential components and optional components of the emulsion composition according to the invention are described in detail.

*<(A) Glycyrrhetic Acid Derivative>*

The emulsion composition according to the invention includes the glycyrrhetic acid derivative represented by Formula (1) above. In the invention, the glycyrrhetic acid derivative can be included in the emulsion composition as an oil-soluble medicinal agent.

The glycyrrhetic acid derivative in the invention is represented by the above Formula (1), and encompasses the following two aspects: i) an aspect in which a hydroxy group at the 3-position of glycyrrhetic acid is esterified with lauric acid, myristic acid, palmitic acid, stearic acid, or eicosanic acid; and ii) an aspect in which a carboxy group at the 20-position of glycyrrhetic acid is esterified with lauryl alcohol, myristyl alcohol, cetyl alcohol, stearyl alcohol, or eicosanol.

The glycyrrhetic acid derivative in the invention is preferably a glycyrrhetic acid derivative in which, in Formula (1), $R^1$ represents $-C_{16}H_{33}$, $-C_{18}H_{37}$, or $-C_{20}H_{41}$ and $R^2$ represents a hydrogen atom; or a glycyrrhetic acid derivative in which $R^1$ represents a hydrogen atom and $R^2$ represents $-(C{=}O)C_{15}H_{31}$ or $-(C{=}O)C_{17}H_{35}$. The glycyrrhetic acid derivative is more preferably stearyl glycyrrhetinate in which $R^1$ represents $-C_{18}H_{37}$ and $R^2$ represents a hydrogen atom. Stearyl glycyrrhetinate, a compound with CAS No. 13832-70-7, is a lipid-soluble substance and has low compatibility with a

general oil agent.

Stearyl glycyrrhetinate has until now been used as an anti-inflammatory agent or an anti-allergic agent for soaps, creams, or milky lotions, but hardly applied to a highly transparent emulsion composition for the above reason.

The method for producing stearyl glycyrrhetinate is not particularly limited. Stearyl glycyrrhetinate can be obtained, for example, by hydrolyzing glycyrrhizic acid extracted from the licorice to produce glycyrrhetic acid, and esterifying the product with stearyl alcohol.

The content of the glycyrrhetic acid derivative in the emulsion composition according to the invention is from 1.0% by mass to 5.0% by mass. In order to produce the desired effect expected from the glycyrrhetic acid derivative sufficiently, the lower limit of the content of the glycyrrhetic acid derivative is preferably 1.5% by mass or more. In terms of tactility of the cosmetic and solubility, the upper limit of the content of the glycyrrhetic acid derivative is 5% by mass.

The content of the glycyrrhetic acid derivative in the emulsion composition according to the invention is preferably from 2.0% by mass to 4.0% by mass. When the content of the glycyrrhetic acid derivative is within the above range, the emulsion composition stably retains high transparency over the long term while it contains the glycyrrhetic acid derivative in high concentration. In a case in which the highly transparent emulsion composition according to the invention is applied to a cosmetic, one of the preferable applications thereof, the cosmetic has excellent stability over time while it contains the glycyrrhetic acid derivative in high concentration, and has improved feeling upon use as a result of relative reduction in the content of the oil agent that may cause deterioration of feeling upon use such as sticky feeling.

*<(B) Specific Oil Agent>*

The emulsion composition according to the invention includes at least one oil agent (specific oil agent) selected from the group consisting of tocopherol, tocopherol acetate and tocotrienol.

As described above, it is preferable to reduce the particle diameter of the dispersed particles contained in the emulsion composition in order to reduce turbidity and reduce the content of the oil agent having a higher refractive index than that of water in order to reduce the particle number.

Accordingly, as an oil agent preferably used in the invention, it is necessary to select an oil agent capable of dissolving a high concentration of glycyrrhetic acid derivative and having a low refractive index.

With regard to the characteristics thereof, the specific oil agents are classified broadly into the following oil agent I and oil agent II according to the relationship between the ability to dissolve the glycyrrhetic acid derivative and the refractive index.

Oil agent I: an oil agent that has excellent ability to dissolve the glycyrrhetic acid derivative and has a higher refractive index than oil agent II.

Oil agent II: an oil agent that has poor ability to dissolve the glycyrrhetic acid derivative compared to oil agent I and has a low refractive index.

In a case in which oil agent I which has excellent ability to dissolve the glycyrrhetic acid derivative is used as the specific oil agent, reduction in turbidity is expected since the content of the oil agent in the emulsion composition can be reduced.

On the other hand, in a case in which oil agent II which has a low refractive index itself is used as the specific oil agent, reduction in turbidity is expected.

In the invention, tocopherol, tocopherol acetate and tocotrienol correspond to oil agent I; and the fatty acid ester having from 10 to 18 carbon atoms corresponds to oil agent II (not falling under the scope of the present invention).

The emulsion composition contains oil agent I as the specific oil agent, or may contain both oil agent I and oil agent II. Each of oil agent I and oil agent II may include a single kind of compound, or may include two or more kinds of compounds in combination.

Since the content of the glycyrrhetic acid derivative is from 1.0% by mass to 5.0% by mass, only tocopherol, tocopherol acetate and tocotrienol as an oil agent belonging to oil agent I as the specific oil agent are used. In a case in which the content of the glycyrrhetic acid derivative is from 0.5% by mass or more and less than 1.0% by mass (not falling under the scope of the present invention), it is preferable to use only an oil agent belonging to oil agent II as the specific oil agent.

*<<Tocopherol and Tocopherol Derivative>>*

Tocopherol and the tocopherol derivative are the specific oil agents belonging to the above-described oil agent I. Tocopherol and the tocopherol derivative are oil agents that have a higher refractive index compared to a fatty acid ester having from 10 to 18 carbon atoms described below, but have extremely-high ability to dissolve the glycyrrhetic acid derivative.

Tocopherol and the tocopherol derivative refer to tocopherol and a tocopherol derivative having a tocopherol skeleton as a basic structure, respectively, and specific examples thereof included according to the present invention

are tocopherol isomers such as α-tocopherol, β-tocopherol, γ-tocopherol, or δ-tocopherol, tocopherol acetate in which a hydroxy group of tocopherol is modified with a fatty acid, and tocotrienol having a tocol skeleton as a basic structure.

The origin of tocopherol and the tocopherol derivative is not particularly limited, and specifically may be sunflower, maize, olive, rapeseed, soybean, peanut, or almond.

According to the present invention, the oil agent is selected from the group consisting of tocopherol, tocopherol acetate, and tocotrienol.

Tocopherol and the tocopherol derivative may be used singly, or in combination of two or more kinds thereof.

*<Fatty Acid Ester Having from 10 to 18 Carbon Atoms>*

The fatty acid ester having from 10 to 18 carbon atoms is the specific oil agent belonging to the above-described oil agent II. The fatty acid ester having from 10 to 18 carbon atoms is an oil agent that has poor ability to dissolve the glycyrrhetic acid derivative compared to tocopherol and the tocopherol derivative, but has a low refractive index.

Examples of a fatty acid as a component of the fatty acid ester having from 10 to 18 carbon atoms include capric acid, sebacic acid, lauric acid, myristic acid, pentadecylic acid, palmitic acid, palmitoleic acid, margaric acid, stearic acid, oleic acid, vaccenic acid, linoleic acid, linolenic acid, and eleostearic acid. Preferable examples thereof include myristic acid, palmitic acid, and sebacic acid. Examples of alcohol reacted with the fatty acid include primary alcohols such as methanol, ethanol, propanol, butanol, or pentanol, and polyhydric alcohols such as ethylene glycol, butylene glycol, propylene glycol, or glycerol. The alcohol may be a linear alcohol or a branched alcohol.

In terms of ability to dissolve the glycyrrhetic acid derivative and low refractive index, the fatty acid ester having from 10 to 18 carbon atoms is preferably isopropyl myristate, isopropyl palmitate, diisopropyl sebacate, propylene glycol caprate, or propylene glycol dicaprate, and more preferably isopropyl myristate, isopropyl palmitate, or diisopropyl sebacate.

Since the content of the glycyrrhetic acid derivative is from 1.0% by mass to 5.0% by mass, the specific oil agent in the invention includes at least one selected from the group consisting of tocopherol, tocopherol acetate, and tocotrienol.

In a case not falling under the scope of the present invention, in which the content of the glycyrrhetic acid derivative is from 0.5% by mass or more and less than 1.0% by mass, it is preferable that the specific oil agent includes at least one selected from the group consisting of isopropyl myristate, isopropyl palmitate, diisopropyl sebacate, propylene glycol caprate, and propylene glycol caprate.

The emulsion composition according to the invention may include an additional oil agent other than the specific oil agent of component (B).

The additional oil agent that can be used in the invention is not particularly limited, as long as it is an oil agent other than component (B). Examples of the additional oil agent that can be used include: at least one selected from the group consisting of an amino-acid ester and a fatty acid glyceride (i.e., component (E) described below); carotenoids; radical scavengers such as an oil-soluble vitamin; liquid oils; fatty acid esters; higher fatty acids; and higher alcohols.

*<(E) Fatty Acid Glyceride>*

Since the content of the glycyrrhetic acid derivative is from 1.0% by mass to 5.0% by mass, (E) which is one or more kinds of fatty acid glycerides is included as the additional oil agent described above in terms of transparency and stability over time of the emulsion composition.

The emulsion composition may additionally include only one or more kinds of amino-acid esters.

*<<Amino-acid Ester>>*

The amino-acid ester is preferably a ester having a glutamic acid skeleton such as dihexyldecyl lauroyl glutamate, diisostearyl lauroyl glutamate, dioctyldodecyl lauroyl glutamate, bis(hexyldecyl/octyldodecyl) lauroyl glutamate, dioctyldodecyl stearoyl glutamate, di(cholesteryl/octyldodecyl) lauroyl glutamate, di(cholesteryl/behenyl/octyldodecyl) lauroyl glutamate, di(phytosteryl/octyldodecyl) lauroyl glutamate, di(octyldodecyl/phytosteryl/behenyl) lauroyl glutamate, (phytosteryl/behenyl/octyldodecyl/isostearyl) lauroyl glutamate, dioctyldodeceth-2 lauroyl glutamate, dioctyldodeceth-5 lauroyl glutamate, disteareth-2 lauroyl glutamate, or disteareth-5 lauroyl glutamate, and more preferably dihexyldecyl lauroyl glutamate, diisostearyl lauroyl glutamate, dioctyldodecyl lauroyl glutamate, bis(hexyldecyl/octyldodecyl) lauroyl glutamate, dioctyldodecyl stearoyl glutamate, or di(cholesteryl/octyldodecyl) lauroyl glutamate.

*<<Fatty Acid Glyceride>>*

The fatty acid glyceride in the invention is preferably mono-, di- ,or triglyceride in which fatty acid is esterified

with glycerol and in which a fatty acid moiety thereof is derived from a liner or branched fatty acid having from 6 to 18 carbon atoms.

Preferable examples of the fatty acid glyceride include triethylhexanoin, glyceryl tricaprylate, glyceryl tricaprate, glyceryl tri(caprylate/caprate), glyceryl trilaurate, glyceryl trimyristate, glyceryl tripalmitoleate, glyceryl tripalmitate, glyceryl trilinoleate, glyceryl trilinolenate, glyceryl tristearate, glyceryl trioleate, and glyceryl triisostearate.

Among these fatty acid glycerides, in terms of stability of the emulsion composition, glyceryl trilinoleate, glyceryl trilinolenate, glyceryl tristearate, glyceryl trioleate, glyceryl triisostearate, glyceryl tricaprylate, glyceryl tricaprate, and glyceryl tri(caprylate/caprate) are preferable.

In the invention, animal fat and oil or plant fat and oil containing the fatty acid glyceride may be used. Examples thereof include camellia Japonica seed oil, macadamia nut oil, castor oil, avocado oil, evening primrose oil, turtle oil, mink oil, egg-yolk oil, persic oil, wheat germ oil, camellia kissi oil, linseed oil, cotton seed oil, perilla oil, tea seed oil, torreya nucifera oil, rice bran oil, china paulownia oil, Japanese tung oil, jojoba oil, germ oil, salad oil, safflower (Carthamus tinctorius) oil, palm oil, coconut oil, peanut oil, almond oil, hazelnut oil, and grape seed oil.

The total content of the specific oil agent of component (B) and the additional oil agent is from 10% by mass to 25% by mass with respect to the total mass of the emulsion composition, in terms of reducing the turbidity of the emulsion composition according to the invention and, when the emulsion composition is applied to the cosmetic, improving feeling upon use of the cosmetic.

Since the content of the glycyrrhetic acid derivative is from 1.0% by mass to 5.0% by mass, the total content of component (B) as the specific oil agent and component (E) as the additional oil agent is preferably from 15% by mass to 20% by mass.

*<(C) Phospholipid>*

The emulsion composition according to the invention include a phospholipid.

Examples of the phospholipid that can be used in the invention include a glycerophospholipid. Examples of the glycerophospholipid include phosphatidic acid, bisphosphatidic acid, a lecithin (phosphatidylcholine), phosphatidylethanolamine, phosphatidylmethylethanolamine, phosphatidylserine, phosphatidylinositol, phosphatidylglycerol, and diphosphatidylglycerol (cardiolipin). Examples of the phospholipid further include those containing the above phospholipid and derived from the plant such soy bean, maize, peanut, rapeseed, or wheat; those containing the above phospholipid and derived from the animal such egg-yolk or cow; and various lecithins derived from the microorganism such as Escherichia coli.

As the glycerophospholipid in the invention, an enzymatic decomposition product of a glycerophospholipid can be used. For example, lysolecithin (enzymatically decomposed lecithin) that is an enzymatic decomposition product of lecithin can be obtained by hydrolyzing lecithin in the presence of an acid or alkaline catalyst or by hydrolyzing lecithin using phospholipase A1 or A2. Examples of the compound name representing the lyso-compound as typified by lysolecithin include lysophosphatidic acid, lysophosphatidylglycerol, lysophosphatidylinositol, lysophosphatidylethanolamine, lysophosphatidylmethylethanolamine, lysophosphatidylcholine (lysolecithin), and lysophosphatidylserine.

The phospholipid in the invention may be used singly, or in mixture of two or more kinds thereof. In terms of transparency and stability over time of the emulsion composition, the phospholipid is preferably a phospholipid other than lysolecithin, and more preferably a purified lecithin.

In a case in which the emulsion composition contains such a lecithin, the transparency of the emulsion composition is further improved, and the transparency, stability over time, and feeling upon use of the cosmetic containing the emulsion composition are further improved.

In the emulsion composition according to the invention, the content of the phospholipid is preferably from 0.1% by mass to 10% by mass, more preferably from 0.2% by mass to 8% by mass, and still more preferably from 1% by mass to 4% by mass, with respect to the total mass of the emulsion composition. In a case in which the content of the phospholipid is 0.1% by mass or more, the emulsion stability of the emulsion composition tends to be further improved. In a case in which the content of the phospholipid is 10% by mass or less, generation of a phospholipid dispersion caused by the dissociation of excess phospholipids from the oil component is suppressed, which is preferable since the emulsion stability of the emulsion composition can be obtained.

Since the content of the glycyrrhetic acid derivative is from 1.0% by mass to 5.0% by mass, the content of the phospholipid as component (C) is preferably from 0.01 to 0.5, more preferably from 0.05 to 0.25, and still more preferably from 0.07 to 0.2, in terms of the content ratio of component (C) to the total content of component (B) and component (E) [C/(B+E)].

*<(D) Water-soluble Surfactant>*

The emulsion composition according to the invention includes a water-soluble surfactant.

The water-soluble surfactant in the invention is preferable since it can significantly decrease the interfacial tension between the oil phase and the aqueous phase of the emulsion composition and as a result, reduce the particle diameter of the emulsion composition.

In terms of stability over time, the water-soluble surfactant in the invention is a surfactant having preferably an HLB of 8 or more, more preferably an HLB of 10 or more, and still more preferably an HLB of 12 or more. The upper limit of the HLB value is not particularly limited, and generally 20 or less, preferably 18 or less.

The HLB used herein represents hydrophilic-hydrophobic balance generally used in the field of surfactants, and can be calculated by using generally used calculation formula such as Kawakami's equation. In the invention, the following Kawakami's equation is employed.

$$HLB = 7 + 11.7 \log (Mw/Mo)$$

In the above equation, Mw represents the molecular weight of a hydrophilic group, and Mo represents the molecular weight of a hydrophobic group.

Alternatively, numerical values of HLB listed in manufacture's catalogs may be used. As is apparent from the above equation, a surfactant having any desired HLB value can be obtained by utilizing the additive property of HLB.

The water-soluble surfactant in the invention is not particularly limited, and examples of thereof include various types of surfactants such as a cationic surfactant, an anionic surfactant, an ampholytic surfactant, and a non-ionic surfactant. Among these, the non-ionic surfactant is preferable. Specific examples of the non-ionic surfactant include a glyceryl fatty acid ester, an organic acid monoglyceride, a polyglyceryl fatty acid ester, a propylene glycol fatty acid ester, a polyglycerol condensed ricinoleic acid ester, a sorbitan fatty acid ester, a sucrose fatty acid ester, and a polyoxyethylene sorbitan fatty acid ester.

The water-soluble surfactant is not necessarily a highly purified product obtained by distillation or the like, and may be a reaction mixture.

The water-soluble surfactant is more preferably a polyglyceryl fatty acid ester, a sorbitan fatty acid ester, a sucrose fatty acid ester, or a polyoxyethylene sorbitan fatty acid ester. The water-soluble surfactant used in the invention is still more preferably a sucrose fatty acid ester or a polyglyceryl fatty acid ester.

The polyglyceryl fatty acid ester used in the invention is preferably an ester of a polyglycerol having an average degree of polymerization of 2 or more (preferably 6 to 15, more preferably 8 to 10) and a fatty acid having 8 to 18 carbon atoms (for example, a fatty acid selected from the group consisting of caprylic acid, capric acid, lauric acid, myristic acid, palmitic acid, stearic acid, oleic acid, and linoleic acid).

Preferable examples of the polyglyceryl fatty acid ester include hexaglyceryl monooleate, hexaglyceryl monostearate, hexaglyceryl monopalmitate, hexaglyceryl monomyristate, hexaglyceryl monolaurate, decaglyceryl monooleate, decaglyceryl monostearate, decaglyceryl monopalmitate, decaglyceryl monomyristate, and decaglyceryl monolaurate. Among these, decaglyceryl monooleate (HLB = 12), decaglyceryl monostearate (HLB = 12), decaglyceryl monopalmitate (HLB = 13), decaglyceryl monomyristate (HLB = 14), and decaglyceryl monolaurate (HLB = 16) are more preferable. These polyglyceryl fatty acid esters may be used singly, or in mixture of two or more kinds thereof.

The sucrose fatty acid ester used in the invention is preferably one having 12 or more, more preferably from 12 to 20 carbon atoms in the fatty acid moiety. Preferable examples of the sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. Among these, sucrose monooleate, sucrose monostearate, sucrose monopalmitate, sucrose monomyristate, and sucrose monolaurate are more preferable. In the invention, these sucrose fatty acid esters may be used singly, or in mixture of two or more kinds thereof.

The sorbitan fatty acid ester used in the invention is preferably one having 8 or more, more preferably 12 or more carbon atoms in the fatty acid moiety. Preferable examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate. These sorbitan fatty acid esters may be used singly, or in mixture of two or more kinds thereof.

The polyoxyethylene sorbitan fatty acid ester used in the invention is preferably one having 8 or more, more preferably 12 or more carbon atoms in the fatty acid moiety. The length (addition mole number) of the ethyleneoxide moiety of the polyoxyethylene is preferably 2 to 100, and more preferably 4 to 50. Preferable examples of the polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate, and polyoxyethylene sorbitan trioleate.

The water-soluble surfactant may be used singly, or in mixture of two or more kinds thereof.

The content of the water-soluble surfactant is preferably from 0.5% by mass to 30% by mass, more preferably from 1% by mass to 20% by mass, and still more preferably from 2% by mass to 15% by mass, with respect to the total mass of the emulsion composition. The water-soluble surfactant content of 0.5% by mass or more is preferable since the solubility of a given amount of glycyrrhetic acid derivative can be improved and the interfacial tension between the oil phase and the aqueous phase can be easily reduced. Furthermore, the water-soluble surfactant content of 30% by mass or less is preferable since the use of an excessive amount of surfactant can be avoided and the foaming of the emulsion composition van be suppressed.

The emulsion composition according to the invention includes the above-described components (A) to (E).

Since the emulsion composition according to the invention includes component (E), the preferable combination of components (A) to (E) is as follows: component (A) is stearyl glycyrrhetinate; component (B) is at least one selected from tocopherol, tocopherol acetate and tocotrienol; component (C) is lecithin; component (D) is at least one selected from a sucrose fatty acid ester or a polyglyceryl fatty acid ester; and component (E) is a fatty acid glyceride.

When components (A) to (E) are combined as above, the emulsion composition with further improved transparency and stability over time, and excellent feeling upon use when blended into cosmetics can be provided.

*<Polyhydric Alcohol>*

It is preferable that the emulsion composition according to the invention includes a polyhydric alcohol in terms of transparency, stability over time, and an antiseptic activity.

The polyhydric alcohol has a moisturizing function, a viscosity adjusting function, and the like. The polyhydric alcohol also has a function for facilitating the extension of the interface by reducing the interfacial tension between water and the oil component, and the formation of fine and stable particles. Therefore, the emulsion composition containing the polyhydric alcohol is preferable, since the particle diameter of the emulsified particles can be further reduced, and the emulsion composition can be maintained stably over the long term while the particle diameter thereof being kept small. Furthermore, the addition of the polyhydric alcohol can reduce the water activity of the emulsion composition and suppress development of microorganisms.

The polyhydric alcohol used in the invention is not particularly limited as long as it is a di- or polyhydric alcohol. Examples of the polyhydric alcohol include glycerol, diglycerol, triglycerol, polyglycerol, 3-methyl-1,3-butanediol, 1,3-butylene glycol, isoprene glycol, polyethylene glycol, 1,2-pentanediol, 1,2-hexanediol, propylene glycol, dipropylene glycol, ethylene glycol, diethylene glycol, and neopentyl glycol. These polyhydric alcohols may be used singly, or in mixture of two or more kinds thereof.

As the polyhydric alcohol, a polyhydric alcohol having three or more hydroxy groups in one molecule thereof is preferably used. By using such a polyhydric alcohol, the interfacial tension between the aqueous medium and the oil component can be reduced more effectively, and a finer and stable emulsion composition can be formed. As a result, in a case in which the emulsion composition according to the invention is applied to cosmetics, percutaneous absorbability can be improved.

Among the polyhydric alcohols satisfying the above-described conditions, glycerol is preferably used since the particle diameter of the emulsion composition can be further reduced and the emulsion composition can be maintained stably over the long term while the particle diameter thereof being kept small.

In terms of transparency exhibited by the emulsion composition, stability over time, and an antiseptic activity, and the viscosity of the emulsion composition, the content of the polyhydric alcohol is preferably from 10% by mass to 60% by mass, more preferably from 20% by mass to 55% by mass, and still more preferably from 30% by mass to 50% by mass, with respect to the total mass of the composition.

The polyhydric alcohol content of 10% by mass or more is preferable since sufficient preservation stability can be easily obtained irrespective of the type, content, or the like of the oil component. Further, the polyhydric alcohol content of 60% by mass or less is preferable since the maximum effect can be obtained and the increase in the viscosity of the emulsion composition can be easily prevented.

*<Other Additives>*

In addition to the above-described components, other additives conventionally used in the field of food, cosmetics, or the like may be appropriately included in the emulsion composition of the invention in accordance with the form thereof.

The additive can be included in the emulsion composition according to the invention as an oil-soluble or water-soluble additive depending on the property thereof.

Examples of the additive include monosaccharides and polysaccharides such as glucose, sucrose, kappa carrageenan, locust bean gum, guar gum, hydroxypropyl guar gum, xanthan gum, karaya gum, tamarind seed polysaccharide, gum arabic, gum tragacanth, hyaluronic acid, sodium hyaluronate, sodium chondroitin sulfate, or dextrin; sugar

alcohols such as sorbitol, mannitol, maltitol, lactose, maltotriitol, or xylitol; inorganic salts such as sodium chloride or sodium sulfate; proteins having a molecular weight exceeding 5000 such as casein, albumin, methylated collagen, hydrolyzed collagen, water-soluble collagen, or gelatin; amino acids and derivatives thereof such as glycine, alanine, valine, leucine, isoleucine, serine, threonine, aspartic acid, glutamic acid, cysteine, methionine, lysine, hydroxylysine, arginine, histidine, phenylalanine, tyrosine, tryptophan, proline, hydroxyproline, or acetylhydroxyproline; synthetic polymers such as a carboxyvinyl polymer, sodium polyacrylate, polyvinyl alcohol, polyethylene glycol, or a block copolymer of ethylene oxide and propylene oxide; and derivatives of water-soluble celluloses such as hydroxyethyl cellulose or methyl cellulose, flavonoids (catechin, anthocyanin, flavone, isoflavone, flavan, flavanone, rutin), ascorbic acid and derivatives thereof (such as ascorbic acid, sodium ascorbate, potassium ascorbate, calcium ascorbate, L-ascorbate phosphate, magnesium ascorbyl phosphate, sodium ascorbyl phosphate, ascorbyl sulfate, disodium ascorbyl sulfate, or ascorbyl-2-glucoside), tocotrienol and derivatives thereof, phenolic acids (chlorogenic acid, ellagic acid, gallic acid, propyl gallate), lignans, crucumins, coumarins, and hydroxy stilbenes such as pterostilbene. The additive may be included as a functional component, a filler, a viscosity adjuster, or a radical scavenger in accordance with the function thereof.

In the invention, other additives such as various medicinal components, a pH adjuster, a pH buffering agent, an ultraviolet absorber, an antiseptic agent, a fragrance, or a colorant that are conventionally used for its purpose can be used in combination.

*<Use Application>*

The emulsion composition according to the invention may be applied to cosmetics and the like. Examples of the cosmetics include a lotion, an essence, a milky lotion, a cream pack or mask, a pack, a hair washing cosmetic, a fragrance cosmetic, a liquid body wash, a UV-care cosmetic, a deodorant cosmetic, and an oral-care cosmetic (in the case of the cosmetic). In particular, the emulsion composition according to the invention is preferably applied to aqueous cosmetics in which is transparency is required.

The lotion and the essence are generally required to have transparency and also required to be transparent even when they deteriorate with age. Here, the above-mentioned turbidity is used as an index of "transparency". Transparency is defined by the absorbance measured with light having a wavelength of 650 nm at 25°C. The absorbance (turbidity) as an index of "transparency" of the cosmetic according to the invention is measured with the above-mentioned spectrometer.

The preferable aspect of the cosmetic prepared by applying the emulsion composition according to the invention is an cosmetic (a cosmetic according to the invention) that includes the emulsion composition according to the invention and has an absorbance (turbidity) measured with light having a wavelength of 650 nm of from 0.001 to 0.2. The absorbance (turbidity) is more preferably from 0.001 to 0.175, and still more preferably from 0.001 to 0.15.

In terms of transparency and stability over time, the cosmetic according to the invention preferably contains at least one selected from non-hydrogenated phospholipids.

In terms of reducing turbidity of the cosmetic according to the invention, it is preferable to reduce the amount of the emulsion composition to be blended into the cosmetic. In this regard, in the cosmetic according to the invention, the amount of the emulsion composition according to the invention to be blended is preferably 10% by mass or less, more preferably 5% by mass or less, and still more preferably 2% by mass or less, with respect to the total mass of the cosmetic.

*[Production Method of Emulsion Composition]*

The method of producing the emulsion composition according to the invention is not particularly limited, and the emulsion composition may be produced in accordance with the conventional method. Example of the preferable method to obtain an emulsion composition include a method including the steps of (a) dissolving a water-soluble surfactant in an aqueous medium to obtain an aqueous phase; (b) mixing the specific oil agent, a phospholipid, and as needed, another oil agent to dissolve and obtain an oil phase; and (c) mixing the aqueous phase and the oil phase under stirring to emulsify and disperse.

The ratio (mass ratio) of the oil phase to the aqueous phase in emulsification dispersion is not particularly limited, and is preferably from 0.1/99.9 to 50/50, more preferably from 0.5/99.5 to 30/70, and still more preferably from 1/99 to 20/80 in terms of the oil phase/aqueous phase ratio (% by mass). The oil phase/aqueous phase ratio of 0.1/99.9 or higher is preferable since the content of the active ingredient is not lowered, and therefore, the practical problem tends not to be caused in the emulsion composition. Furthermore, the oil phase/aqueous phase ratio of 50/50 or smaller is preferable since the surfactant concentration is not lowered and stability over time of the emulsion composition is maintained.

In the emulsification dispersion, the emulsification operation may be performed in one-step, and is preferably performed in two-steps in order obtain uniform and fine emulsion particles. More specifically, it is preferable to employ

two or more emulsifying apparatuses, for example, in an emulsification method in which a one-step emulsification operation is performed using a conventional emulsifying device utilizing shearing force (such as a stirrer, an impeller, a homomixer, or a continuous flow shear apparatus), and then emulsification is performed by passing through a high-pressure homogenizer, an ultrasonic disperser, or the like. The finely emulsified droplets having a more uniform particle size can be obtained by using the high-pressure homogenizer. The emulsification operation may be performed plural times in order to obtain droplets having a still more uniform particle size.

In the emulsion composition according to the invention, an pH adjuster may be used appropriately. The pH value of the emulsion composition according to the invention in which pH is adjusted by the pH adjuster is preferably is a range of from 4 to 10.

The pH adjuster may be used prior to the preparation of the emulsion composition, or after the preparation of the emulsion composition. It is preferable to add the pH adjuster prior to the preparation of the emulsion composition, since the range of pH change can be reduced and pH can be adjusted under a mild condition. In this case, the pH adjuster may be added to an aqueous phase composition or an oil phase composition prior to the preparation of the emulsion composition.

Examples of the pH adjuster that can be used include an inorganic acid, an organic acid, an inorganic base, and a salt thereof. Specific examples of the pH adjuster include, but not limited thereto, organic acids and salts thereof such as critic acid, gluconic acid, carboxylic acid, tartaric acid, succinic acid, acetic acid, phthalic acid, trifluoroacetic acid, morpholineethanesulfonic acid, or 2-[4-(2-hydroxyethyl)-1-piperazinyl]ethanesulfonic acid; inorganic bases such as tris(hydroxymethyl) aminomethane, or ammonia; inorganic acids and salts thereof such as hydrochloric acid, perchloric acid, or carbonic acid; inorganic bases such as sodium phosphate, potassium phosphate, calcium hydroxide, sodium hydroxide, potassium hydroxide, or magnesium hydroxide; and inorganic hydrogen salts such as sodium hydrogenphosphate or sodium hydrogencarbonate.

The cosmetic according to the invention can be obtained, for example, by mixing the emulsion composition according to the invention, and a component that may be added as needed using a stirrer, an impeller, or homomixer.

## EXAMPLES

Hereinafter, the invention is described more specifically with reference to the examples. However, the invention is not limited to these examples.

### *1. Preparation and Evaluation of Emulsion Composition*

*[Example 1-1] for reference*

*<Preparation of Emulsion Composition (EM-1)>*

The following components were dissolved under heating at 70°C for 1 hour to obtain an aqueous phase composition.

- Sucrose monostearate (HLB = 16) 9.3 g
- Decaglyceryl monooleate (HLB = 12) 18.9 g
- Glycerol 125.97 g
- Pure water 83.97 g

The following components were dissolved under heating at 70°C for 1 hour to obtain an oil phase composition.

- Stearyl glycyrrhetinate 2.76 g
- Tocopherol 27.81 g
- Glyceryl tri(caprylate/caprate) 27.69 g
- Purified lecithin (from soybean) 3.6 g

While stirring the aqueous phase composition, the oil phase composition was added thereto. The mixture was then dispersed for 4 minutes using a high-pressure homogenizer, thereby obtaining a preliminary emulsion.

The obtained preliminary emulsion was cooled to approximately 60°C, and subjected to high-pressure emulsification under a pressure of 245 MPa using Altimizer HJP-25005 (manufactured by Sugino Machine Limited).

Subsequently, the obtained emulsion composition was filtered through a microfilter of 1 μm in average pore size, and the filtrate was sterilized by heating at 80°C for 30 minutes, thereby preparing a glycyrrhetic acid derivative containing emulsion composition EM-1.

The details of the respective components used for the preparation of the aqueous phase composition and the oil phase composition are described below.

*[Examples 1-2 to 1-30 including Reference Examples 1-2, 1-3, 1-11 to 1-15 and 1-30, Comparative Examples 1-1 to 1-5]*

All of glycyrrhetic acid derivative containing emulsion compositions EM-2 to EM-35 of Examples 1-2 to 1-30 (including Reference Examples 1-2, *1-3, 1-11 to 1-15 and 1-30)* and Comparative Examples 1-1 to 1-5 were obtained in the same manner as Reference Example 1-1, except that the kind and the amount of each of the components used for preparation of the glycyrrhetic acid derivative containing emulsion composition EM-1 in Reference Example 1-1 were changed as shown in Tables 1 to 4.

The glycyrrhetic acid derivative containing emulsion composition EM-11 of Comparative Examples 1-1 is an emulsion composition in which the content of the glycyrrhetic acid derivative is lower than the lower limit of the content of the glycyrrhetic acid derivative in the emulsion composition according to the invention.

*[Evaluation of Emulsion Composition]*

Each of the glycyrrhetic acid derivative containing emulsion compositions obtained above was evaluated with respect to an initial turbidity and stability over time. The details are as follows.

*1-1. Evaluation of Initial Turbidity*

0.1 g each of the glycyrrhetic acid derivative containing emulsion compositions (EM-1 to EM-35) was added to 9.9 g of pure water, followed by stirring for 10 minutes using a magnetic stirrer, thereby obtaining a water-diluted solution.

With respect to each of the water-diluted solutions of the emulsion compositions, the absorbance at 650 nm as an index of turbidity was measured at 25°C using a spectrometer (V-630, manufactured by JASCO Corporation). The initial turbidity was evaluated in accordance with the following evaluation criteria.

*Evaluation Criteria*

S: 0.1 or less

A: more than 0.1 and 0.15 or less

B: more than 0.15 and 0.2 or less

C: more than 0.2

*1-2. Evaluation of Stability Over Time*

With regard to the evaluation of stability over time (durability of transparency), the absorbance of each of the glycyrrhetic acid derivative containing emulsion compositions stored for 1 year at 4°C was measured in the same manner as the initial turbidity measurement. The rate of change A (%) in absorbance from the initial turbidity measured immediately after the preparation was determined based on the following equation, and evaluated based on the following criteria.

$$\text{Rate of change A (\%)} = \text{absorbance measured after storing for 1 year at } 4°\text{C/absorbance measured immediately after the preparation} \times 100$$

A rate of change A (%) closer to 100% indicates excellent stability over time of the emulsion composition.

*Evaluation Criteria*

S: less than 120%

A: 120% or more and less than 150%
B: 150% or more and less than 200%
C: 200% or more

The results are shown in the following Tables 1 to 4.

Table 1

| | | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 | Ex. 1-10 | Comp. Ex. 1-1 |
|---|---|---|---|---|---|---|---|---|---|---|---|---|---|
| No. of emulsion composition | | | EM-1 | EM-2 | EM-3 | EM-4 | EM-5 | EM-6 | EM-7 | EM-8 | EM-9 | EM-10 | EM-11 |
| Composition | Component (A) | Stearyl glycyrrhetinate | 0.92 | 0.5 | 0.92 | 3.38 | 3.38 | 3.38 | 3.38 | 3.38 | 3.38 | 3.38 | 0.2 |
| | Component (B) | Tocopherol | 9.27 | — | 9.27 | 15.76 | 14.43 | 14.43 | 14.43 | 14.43 | 14.43 | 14.43 | — |
| | | Tocopherol acetate | — | — | — | — | 1.33 | — | — | — | — | — | — |
| | | Tocotrienol | — | — | — | — | — | 1.33 | — | — | — | — | — |
| | | Isopropyl myristate | — | 24.5 | — | — | — | — | 1.33 | — | — | — | 24.5 |
| | | Isopropyl palmitate | — | — | — | — | — | — | — | 1.33 | — | — | — |
| | | Diisopropyl sebacate | — | — | 9.23 | — | — | — | — | — | — | — | — |
| | | Propylene glycol caprate | — | — | — | — | — | — | — | — | 1.33 | — | — |
| | | Propylene glycol dicaprate | — | — | — | — | — | — | — | — | — | 1.33 | — |
| | Component (C) | Phospholipid: purified lecithin | 1.2 | 1.9 | 1.9 | 1.9 | 1.9 | 1.9 | 1.9 | 1.9 | 1.9 | 1.9 | 1.9 |
| | Component (D) | Decaglyceryl monooleate | 6.30 | 6.30 | 6.30 | 9.40 | 9.40 | 9.40 | 9.40 | 9.40 | 9.40 | 9.40 | 6.30 |
| | | Sucrose monostearate | 3.10 | 3.10 | 3.10 | — | — | — | — | — | — | — | 3.10 |
| | Component (E) | Glyceryl tri(caprylate/caprate) | 9.23 | — | — | 2.74 | 2.74 | 2.74 | 2.74 | 2.74 | 2.74 | 2.74 | — |
| | Other components | Glycerol | 41.99 | 38.22 | 41.57 | 40.09 | 40.09 | 40.09 | 40.09 | 40.09 | 40.09 | 40.09 | 38.4 |
| | | Pure water | Balance | Balance | Balance | Balance | Balance | Balance | Balance | Balance | Balance | Balance | Balance |
| Total (addition amount of each component above is based on % by mass) | | | 100 | 100 | 100 | 100 | 100 | 100 | 100 | 100 | 100 | 100 | 100 |
| Total content of oil agent (B+E, % by mass) | | | 18.50 | 24.50 | 18.50 | 18.50 | 18.50 | 18.50 | 18.50 | 18.50 | 18.50 | 18.50 | 24.50 |
| Evaluation | Transparency | Initial turbidity: evaluation | S | S | S | S | S | S | S | S | S | S | S |
| | | Initial turbidity: absorbance at 650 nm | 0.046 | 0.043 | 0.057 | 0.080 | 0.075 | 0.085 | 0.072 | 0.072 | 0.073 | 0.074 | 0.043 |
| | Stability over time | Store for 1 year at 4°C | S | S | S | S | S | S | S | S | S | S | S |
| | | Rate of change A (%) | 114 | 118 | 107 | 111 | 108 | 110 | 113 | 112 | 116 | 113 | 119 |

In Table 1, Ex. 1-1 to Ex. 1-3 are Reference Examples.

Table 2

| | | | Ex. 1-11 | Ex. 1-12 | Ex. 1-13 | Ex. 1-14 | Ex. 1-15 | Comp. Ex. 1-2 | Comp. Ex. 1-3 | Comp. Ex. 1-4 |
|---|---|---|---|---|---|---|---|---|---|---|
| No. of emulsion composition | | | EM-12 | EM-13 | EM-14 | EM-15 | EM-16 | EM-17 | EM-18 | EM-19 |
| Composition | Component (A) | Stearyl glycyrrhetinate | 0.5 | 0.5 | 3.38 | 3.38 | 3.38 | 3.38 | 0.5 | 0.5 |
| | Component (B) | Tocopherol | 2.25 | — | 14.1 | 14.1 | 14.1 | — | — | — |
| | | Isopropyl myristate | — | 30 | — | — | — | — | — | — |
| | Component (C) | Phospholipid: purified lecithin | 1 | 1.9 | 3.3 | 3.3 | 3.3 | 1.9 | 1.9 | 1.2 |
| | Component (D) | Decaglyceryl monooleate | 6.3 | 6.3 | 9.4 | 9.4 | 9.4 | 6.3 | 6.3 | 6.3 |
| | | Sucrose monostearate | 3.1 | 3.1 | — | — | — | 3.1 | 3.1 | 3.1 |
| | Component (E) | Glyceryl tri(caprylate/caprate) | — | — | — | — | — | — | — | 87.5 |
| | | Dihexyldecyl lauroyl glutamate | — | — | 2.5 | — | — | — | — | — |
| | | Dioctyldodecyl stearoyl glutamate | — | — | — | 2.5 | — | — | — | — |
| | | Di(phytosteryl/octyldodecyl) lauroyl glutamate | — | — | — | — | 2.5 | — | — | — |
| | | Isostearic acid | — | — | — | — | — | — | 12 | — |
| | | Isostearyl alcohol | — | — | — | — | — | — | — | — |
| | | Methylpentanediol dineopentanoate | — | — | — | — | — | 40 | — | — |
| | Other components | Glycerol | 52.1 | 34.92 | 40.39 | 40.39 | 40.39 | 27.19 | 45.72 | 0.84 |
| | | Pure water | Balance | Balance | Balance | Balance | Balance | Balance | Balance | Balance |
| Total (addition amount of each component above is based on % by mass) | | | 100 | 100 | 100 | 100 | 100 | 100 | 100 | 100 |
| Total content of oil agent (B+E, % by mass) | | | 2.3 | 30 | 16.6 | 16.6 | 16.6 | 40 | 12 | 12 |
| Evaluation | Transparency | Initial turbidity: evaluation | S | S | S | S | S | C | A | Non-emulsifiable |
| | | Initial turbidity: absorbance at 650 nm | 0.02 | 0.053 | 0.072 | 0.078 | 0.075 | 0.207 | 0.105 | |
| | Stability over time | Store for 1 year at 4°C | S | A | S | S | S | B | C | |
| | | Rate of change A (%) | 113 | 135 | 107 | 108 | 108 | 183 | 243 | |

In Table 2, Ex. 1-11 to Ex. 1-15 are Reference Examples.

Table 3

| | | | Ex. 1-16 | Ex. 1-17 | Ex. 1-18 | Comp. Ex. 1-5 | Ex. 1-19 | Ex. 1-20 | Ex. 1-21 | Ex. 1-22 | Ex. 1-23 |
|---|---|---|---|---|---|---|---|---|---|---|---|
| No. of emulsion composition | | | EM-20 | EM-21 | EM-22 | EM-23 | EM-24 | EM-25 | EM-26 | EM-27 | EM-28 |
| Composition | Component (A) | Stearyl glycyrrhetinate | 3.38 | 3.38 | 3.38 | 3.38 | 3.38 | 3.38 | 3.38 | 3.38 | 3.38 |
| | Component (B) | Tocopherol | 14.1 | 14.1 | 14.1 | 14.1 | 14.1 | 15.76 | 15.76 | 15.76 | 15.76 |
| | Component (C) | Phospholipid: purified lecithin | 1.9 | 1.9 | 1.9 | 1.9 | 1.9 | 1.9 | — | — | — |
| | | Phospholipid: crude lecithin | — | — | — | — | — | — | 1.9 | — | — |
| | | Phospholipid: lysolecithin | — | — | — | — | — | — | — | 1.9 | — |
| | | Phospholipid: hydrogenated lecithin | — | — | — | — | — | — | — | — | 1.9 |
| | Component (D) | Decaglyceryl monooleate | 9.4 | 6.3 | — | — | — | 6.3 | 6.3 | 6.3 | 6.3 |
| | | Decaglyceryl monomyristate | — | — | — | — | 9.4 | — | 12 | — | — |
| | | Sucrose monostearate | — | 3.1 | 9.4 | — | — | 3.1 | 3.1 | 3.1 | 3.1 |
| | Component (E) | Glyceryl tri(caprylate/caprate) | 2.5 | 2.5 | 2.5 | 2.5 | 2.5 | 2.74 | 2.74 | 2.74 | 2.74 |
| | Other components | Glycerol | 41.23 | 41.23 | 41.23 | 46.87 | 41.23 | 40.09 | 40.09 | 40.09 | 40.09 |
| | | Pure water | Balance | Balance | Balance | Balance | Balance | Balance | Balance | Balance | Balance |
| Total (addition amount of each component above is based on % by mass) | | | 100 | 100 | 100 | 100 | 100 | 100 | 100 | 100 | 100 |
| Total content of oil agent (B+E, % by mass) | | | 16.6 | 16.6 | 16.6 | 16.6 | 16.6 | 18.5 | 18.5 | 18.5 | 18.5 |
| Evaluation | Transparency | Initial turbidity: evaluation | S | S | A | Non-emulsifiable | S | S | A | B | S |
| | | Initial turbidity: absorbance at 650 nm | 0.085 | 0.09 | 0.132 | | 0.098 | 0.086 | 0.102 | 0.198 | 0.071 |
| | Stability over time | Store for 1 year at 4°C | S | S | A | | S | S | S | A | S |
| | | Rate of change A (%) | 110 | 117 | 132 | | 106 | 107 | 113 | 143 | 118 |

Ex.: Example; Comp. Ex.: Comparative Example.

Table 4

| | | | Ex. 1-24 | Ex. 1-25 | Ex. 1-26 | Ex. 1-27 | Ex. 1-28 | Ex. 1-29 | Ex. 1-30 |
|---|---|---|---|---|---|---|---|---|---|
| No. of emulsion composition | | | EM-29 | EM-30 | EM-31 | EM-32 | EM-33 | EM-34 | EM-35 |
| Composition | Component (A) | Stearyl glycyrrhetinate | 3.38 | 3.38 | 3.38 | 3.38 | 3.38 | 3.38 | 3.38 |
| | Component (B) | Tocopherol | 14.1 | 14.1 | 14.1 | 14.1 | 14.1 | 14.1 | 14.1 |
| | Component (C) | Phospholipid: purified lecithin | 1.2 | 2.5 | 3.3 | 5 | 6.6 | 10 | 3.3 |
| | Component (D) | Decaglyceryl monooleate | 6.3 | 6.3 | 9.4 | 9.4 | 9.4 | 9.4 | 9.4 |
| | | Sucrose monostearate | 3.1 | 3.1 | — | — | — | — | — |
| | Component (E) | Glyceryl tri(caprylate/caprate) | 2.5 | 2.5 | 2.5 | 2.5 | 2.5 | 2.5 | — |
| | | Persic oil (main component: glyceryl trioleate) | — | — | — | — | — | — | 2.5 |
| | Other components | Glycerol | 41.65 | 40.87 | 40.39 | 39.37 | 38.41 | 36.37 | 40.39 |
| | | Pure water | Balance | Balance | Balance | Balance | Balance | Balance | Balance |
| Total (all of the above addition amounts are based on % by mass) | | | 100 | 100 | 100 | 100 | 100 | 100 | 100 |
| Total content of oil agent (B+E, % by mass) | | | 16.6 | 16.6 | 16.6 | 16.6 | 16.6 | 16.6 | 16.6 |
| Phospholipid (C)/oil amount (B+E) | | | 0.07 | 0.15 | 0.2 | 0.3 | 0.4 | 0.6 | 0.2 |
| Evaluation | Transparency | Initial turbidity: evaluation | S | S | S | A | A | B | S |
| | | Initial turbidity: absorbance at 650 nm | 0.085 | 0.09 | 0.084 | 0.112 | 0.14 | 0.198 | 0.089 |
| | Stability over time | Store for 1 year at 4°C | S | S | S | S | A | B | S |
| | | Rate of change A (%) | 106 | 118 | 109 | 119 | 132 | 185 | 104 |

In Table 4, Ex. 1-30 is a Reference Example.

The details of the respective components shown in Tables 1 to 4 are as follows.

Sucrose monostearate used is RYOTO SUGAR ESTER S-1670 (HLB = 16) manufactured by Mitsubishi-Kagaku Foods Corporation.

Decaglyceryl monooleate (also known as decaglycerol monooleate) and decaglyceryl monomyristate (also known as decaglycerol monomyristate) are NIKKOL DECAGLYN 1-O (HLB = 12) and NIKKOL DECAGLYN 1-M (HLB = 14) manufactured by Nikko Chemicals Co., Ltd., respectively.

Tocopherol used is RIKEN E-OIL 800 manufactured by RIKEN VITAMIN Co., Ltd. Tocopherol acetate used is VITAMIN E ACETATE manufactured by BASF.

Isopropyl myristate, isopropyl palmitate, diisopropyl sebacate, propylene glycol caprate, and propylene glycol dicaprate used are NIKKOL IPM-100, IPP, DIS, SEFSOL-218, AND SEFSOL-228 manufactured by Nikko Chemicals Co., Ltd., respectively.

Glyceryl tri(caprylate/caprate) used is COCONAD MT manufactured by Kao Corporation.

Isostearic acid and isostearyl alcohol used are ISOSTEARIC ACID EX and ISOSTEARYL ALCOHOL EX LECITHIN manufactured by Kokyu Alcohol Kogyo Co. Ltd., respectively.

Methylpentanediol dineopentanoate used is NEOSOLUE-MP manufactured by Nippon Fine Chemical.

Purified lecithin (SLP-White), crude lecithin (SLP-Paste), lysolecithin (White-Lyso), and hydrogenated lecithin (SLP-White H) used are the products from soybean manufactured by Tsuji Oil Mills Co. Ltd.

*2. Preparation and Evaluation of Cosmetic*

As examples of the cosmetic, lotions and gel cosmetics were prepared using the glycyrrhetic acid derivative containing emulsion compositions obtained above, and evaluated with respect to transparency, stability, and feeling upon use.

*[Examples 2-1 to 2-46 including Reference Examples 2-1, 2-6 to 2-8, 2-16 and 2-17, Comparative Examples 2-1 and 2-2]*

*<Preparation of Lotion>*

Of the glycyrrhetic acid derivative containing emulsion compositions obtained above, each of the emulsion compositions shown in Tables 5 and 6 was diluted with a model solution of a lotion formulation prepared as described below such that the content of the glycyrrhetic acid derivative is from 0.01% by mass to 0.5% by mass. The diluted solution was stirred for 10 minutes using a magnetic stirrer, thereby preparing an evaluation sample.

The content of the glycyrrhetic acid derivative in the lotion of each Example is shown in Tables 5 and 6.

*Preparation of Model Solution of Lotion Formulation*

10% by mass of 1,3-butylene glycol (BG) as polyhydric alcohol and 0.3% by mass of phenoxyethanol as an antiseptic agent were added to 10mM critic acid buffer solution (pH 7) such that the content thereof in the cosmetic composition was as shown in Tables 5 and 6. The resultant was then mixed, thereby preparing a model solution of a lotion formulation.

*[Reference Example 2-17 and Examples 2-18 to 2-21]*

*<Preparation of Gel Cosmetic>*

A carboxyvinyl polymer and an acrylic acid-alkyl methacrylate copolymer as thickeners were added to a purified water in which 1,3-butylene glycol (BG) as polyhydric alcohol, glycerol, and phenoxyethanol as an antiseptic agent. The mixture was then stirred and dispersed, thereby preparing a model solution of a gel formulation. Subsequently, of the glycyrrhetic acid-containing emulsion compositions obtained above, each of the emulsion compositions shown in Table 7 was added to the model solution of a gel formulation such that the content of the glycyrrhetic acid derivative is 0.05% by mass. The mixture was stirred and the pH thereof was adjusted (to pH 6.0) using NaOH as a pH adjuster, thereby preparing a gel cosmetic.

*[Evaluation]*

Each of the lotions and the gel cosmetics obtained above was evaluated with respect to the initial turbidity and stability over time. Furthermore, feeling upon use of the cosmetics was evaluated by dedicated panels. The details are as follows.

*2-1. Evaluation of Initial Turbidity*

With respect to each of the lotions and the gel cosmetics obtained above, the absorbance at 650 nm as an index of turbidity was measured at 25°C using a spectrometer (U-3310, manufactured by Hitachi, Ltd.). The initial turbidity was evaluated in accordance with the following evaluation criteria.

*Evaluation Criteria*

S: 0.1 or less

A: more than 0.1 and 0.15 or less

B: more than 0.15 and 0.2 or less

C: more than 0.2

*2-2. Evaluation of Stability Over Time*

With respect to the lotions and the gel cosmetics obtained above, the change in turbidity from the initial turbidity measured immediately after the preparation to the turbidity measured after storing under the temperature of 40°C was determined to evaluate stability over time (durability of transparency in cosmetics).

With regard to the change in turbidity, the absorbance of each of the lotions and the gel cosmetics stored for 2 weeks at 40°C with respect to light having a wavelength of 650 nm was measured in the same manner as the initial turbidity measurement. The rate of change B (%) in absorbance from the initial turbidity measured immediately after the preparation was determined based on the following equation, and evaluated based on the following criteria.

$$\text{Rate of change B (\%)} = \text{absorbance measured after storing for 2 weeks at 40°C/absorbance measured immediately after the preparation} \times 100$$

A rate of change B (%) closer to 100% indicates excellent stability over time of the cosmetic.

*Evaluation Criteria*

S: less than 120%
A: 120% or more and less than 150%
B: 150% or more and less than 200%
C: 200% or more

*2-3. Evaluation of Feeling upon use*

With respect to the lotions and the gel cosmetics of Examples and Comparative Examples, the quality of feeling upon use (rich feeling, little sticky feeling) was evaluated by 10 dedicated panels based on the following criteria.

*Evaluation Criteria*

S: 7 or more of the 10 panels answered "good"
A: 5 to 6 of the 10 panels answered "good"
B: 3 to 4 of the 10 panels answered "good"
C: 2 or less of the 10 panels answered "good"

The evaluation results are shown in Tables 5 and 6 with regard to the lotions and Table 7 with regard to the gel cosmetics.

Table 5

| | | | Ex. 2-1 | Ex. 2-2 | Ex. 2-3 | Ex. 2-4 | Ex. 2-5 | Ex. 2-6 | Ex. 2-7 | Ex. 2-8 | Comp. Ex. 2-1 | Comp. Ex. 2-2 |
|---|---|---|---|---|---|---|---|---|---|---|---|---|
| Composition | Emulsion composition | EM-3 | 5.4 | — | — | — | — | — | — | — | — | — |
| | | EM-5 | — | 1.5 | — | — | — | — | — | — | — | — |
| | | EM-7 | — | — | 0.3 | 1.5 | 3 | — | — | — | — | — |
| | | EM-11 | — | — | — | — | — | — | — | — | 25 | — |
| | | EM-14 | — | — | — | — | — | 1.5 | — | — | — | — |
| | | EM-15 | — | — | — | — | — | — | 1.5 | — | — | — |
| | | EM-16 | — | — | — | — | — | — | — | 1.5 | — | — |
| | | EM-18 | — | — | — | — | — | — | — | — | — | 10 |
| | Model solution of lotion formulation | BG | 10 | 10 | 10 | 10 | 10 | 10 | 10 | 10 | 10 | 10 |
| | | Phenoxyethanol | 0.3 | 0.3 | 0.3 | 0.3 | 0.3 | 0.3 | 0.3 | 0.3 | 0.3 | 0.3 |
| | | Critic acid buffer (pH 7) | Balance | Balance | Balance | Balance | Balance | Balance | Balance | Balance | Balance | Balance |
| Total (addition amount of each component above is based on % by mass) | | | 100 | 100 | 100 | 100 | 100 | 100 | 100 | 100 | 100 | 100 |
| The content of stearyl glycyrrhetinate in lotion (% by mass) | | | 0.05 | 0.05 | 0.01 | 0.05 | 0.1 | 0.05 | 0.05 | 0.05 | 0.05 | 0.05 |
| Evaluation | Transparency | Initial turbidity: evaluation | B | S | S | S | B | S | A | A | C | C |
| | | Initial turbidity: absorbance at 650 nm | 0.188 | 0.095 | 0.019 | 0.092 | 0.182 | 0.100 | 0.108 | 0.104 | 0.324 | 0.303 |
| | Stability over time | Store for 2 weeks at 40°C | A | S | S | S | S | S | S | S | B | C |
| | | Rate of change B (%) | 130% | 104% | 106% | 104% | 114% | 103% | 103% | 102% | 168% | 211% |
| | Feeling upon use | | A | S | S | S | S | S | S | S | C | B |

In Table 5, Ex. 2-1 and Ex. 2-6 to Ex. 2-8 are Reference Examples.

Table 6

| | | | Ex. 2-9 | Ex. 2-10 | Ex. 2-11 | Ex. 2-12 | Ex. 2-13 | Ex. 2-14 | Ex. 2-15 | Ex. 2-16 |
|---|---|---|---|---|---|---|---|---|---|---|
| Composition | Emulsion composition | EM-22 | 1.5 | — | — | — | — | — | — | — |
| | | EM-26 | — | 1.5 | — | — | — | — | — | — |
| | | EM-27 | — | — | 1.5 | — | — | — | — | — |
| | | EM-28 | — | — | — | 1.5 | — | — | — | — |
| | | EM-29 | — | — | — | — | 1.5 | — | — | — |
| | | EM-31 | — | — | — | — | — | 1.5 | — | — |
| | | EM-33 | — | — | — | — | — | — | 1.5 | — |
| | | EM-35 | — | — | — | — | — | — | — | 1.5 |
| | Model solution of lotion formulation | BG | 10 | 10 | 10 | 10 | 10 | 10 | 10 | 10 |
| | | Phenoxyethanol | 0.3 | 0.3 | 0.3 | 0.3 | 0.3 | 0.3 | 0.3 | 0.3 |
| | | Critic acid buffer (pH 7) | Balance | Balance | Balance | Balance | Balance | Balance | Balance | Balance |
| Total (addition amount of each component above is based on % by mass) | | | 100 | 100 | 100 | 100 | 100 | 100 | 100 | 100 |
| The content of stearyl glycyrrhetinate in lotion (% by mass) | | | 0.05 | 0.05 | 0.05 | 0.05 | 0.05 | 0.05 | 0.05 | 0.05 |
| Evaluation | Transparency | Initial turbidity: evaluation | B | A | B | A | S | A | B | A |
| | | Initial turbidity: absorbance at 650 nm | 0.165 | 0.147 | 0.167 | 0.109 | 0.097 | 0.117 | 0.168 | 0.121 |
| | Stability over time | Store for 2 weeks at 40°C | A | S | S | B | A | S | S | S |
| | | Rate of change B (%) | 128% | 109% | 117% | 175% | 129% | 105% | 104% | 102% |
| | Feeling upon use | | S | S | S | S | S | S | A | S |

In Table 6, Ex. 2-16 is a Reference Example.

Table 7

| | | | Ex. 2-17 | Ex. 2-18 | Ex. 2-19 | Ex. 2-20 | Ex. 2-21 |
|---|---|---|---|---|---|---|---|
| Composition | Emulsion composition | EM-2 | 10 | — | — | — | — |
| | | EM-4 | — | 1.5 | — | — | — |
| | | EM-5 | — | — | 1.5 | — | — |
| | | EM-7 | — | — | — | 1.5 | — |
| | | EM-28 | — | — | — | — | 1.5 |
| | Model solution of gel formulation | BG | 5 | 5 | 5 | 5 | 5 |
| | | Glycerol | 3 | 3 | 3 | 3 | 3 |
| | | Carboxyvinyl polymer | 0.4 | 0.4 | 0.4 | 0.4 | 0.4 |
| | | Acrylic acid-alkyl methacrylate copolymer | 0.2 | 0.2 | 0.2 | 0.2 | 0.2 |
| | | Phenoxyethanol | 0.3 | 0.3 | 0.3 | 0.3 | 0.3 |
| | | NaOH | Adequate amount | Adequate amount | Adequate amount | Adequate amount | Adequate amount |
| | | Purified water | Balance | Balance | Balance | Balance | Balance |
| Total (addition amount of each component above is based on % by mass) | | | 100 | 100 | 100 | 100 | 100 |
| The content of stearyl glycyrrhetinate in gel cosmetic (% by mass) | | | 0.05 | 0.05 | 0.05 | 0.05 | 0.05 |
| Evaluation | Transparency | Initial turbidity: evaluation | A | S | S | S | S |
| | | Initial turbidity: absorbance at 650 nm | 0.148 | 0.09 | 0.088 | 0.086 | 0.109 |
| | Stability over time | Store for 2 weeks at 40°C | A | S | S | S | B |
| | | Rate of change B (%) | 138% | 103% | 105% | 105% | 158% |
| | Feeling upon use | | A | S | S | S | S |

In Table 7, Ex. 2-17 is a Reference Example.

As shown in Tables 1 to 4, it was confirmed that the emulsion compositions of the Examples have high transparency and excellent stability over time, while containing the glycyrrhetic acid derivative in high concentration.

Furthermore, as shown in Tables 5 to 7, it was confirmed that the cosmetics using the emulsion compositions of the Examples have high transparency and excellent stability over time, and give favorable feeling upon use (rich feeling, non-sticky feeling), while containing the glycyrrhetic acid derivative in high concentration.

## Claims

1. An emulsion composition comprising the following components (A) to (E):

   (A) a glycyrrhetic acid derivative represented by the following Formula (1);
   (B) at least one oil agent selected from the group consisting of tocopherol, tocopherol acetate and tocotrienol;
   (C) a phospholipid;
   (D) a water-soluble surfactant; and
   (E) a fatty acid glyceride,

   wherein a content of the glycyrrhetic acid derivative represented by Formula (1) is from 1.0% by mass to 5.0% by mass:

   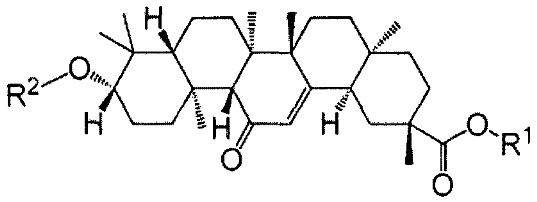

   (1)

   wherein, in Formula (1), with regard to $R^1$ and $R^2$, $R^1$ represents $-C_{12}H_{25}$, $-C_{14}H_{29}$, $-C_{16}H_{33}$, $-C_{18}H_{37}$, or $-C_{20}H_{41}$, and $R^2$ represents a hydrogen atom; or $R^1$ represents a hydrogen atom, and $R^2$ represents $-(C{=}O)C_{11}H_{23}$, $-(C{=}O)C_{13}H_{27}$, $-(C{=}O)C_{15}H_{31}$, or $-(C{=}O)C_{17}H_{35}$, and
   wherein a total content of component (B) and component (E) is from 10% by mass to 25% by mass.

2. The emulsion composition according to claim 1, wherein component (A) comprises stearyl glycyrrhetinate.

3. The emulsion composition according to claim 1 or 2, wherein component (D) comprises at least one selected from the group consisting of a sucrose fatty acid ester and a polyglyceryl fatty acid ester.

4. The emulsion composition according to any one of claims 1 to 3, wherein component (C) comprises a lecithin.

5. The emulsion composition according to any one of claims 1 to 4, wherein a content ratio of component (C) to a total content of component (B) and component (E) [C/(B+E)] is from 0.01 to 0.5 in terms of mass.

6. A cosmetic comprising the emulsion composition according to any one of claims 1 to 5.

7. The cosmetic according to claim 6, wherein an absorbance measured with light having a wavelength of 650 nm is from 0.001 to 0.2.

## Patentansprüche

1. Emulsionszusammensetzung enthaltend die folgenden Komponenten (A) bis (E):

   (A) ein durch die folgende Formel (1) dargestelltes Glycyrrethinsäurederivat;
   (B) mindestens ein Ölagens, ausgewählt aus der Gruppe bestehend aus Tocopherol, Tocopherolacetat und Tocotrienol;
   (C) ein Phospholipid;
   (D) ein wasserlösliches Tensid; und
   (E) ein Fettsäureglycerid,

wobei ein Gehalt an dem durch Formel (1) dargestellten Glycyrrethinsäurederivat 1,0 Masse% bis 5,0 Masse% beträgt:

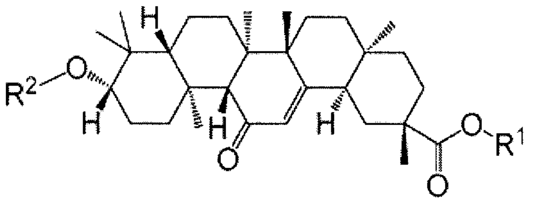

(1)

wobei in Formel (1), im Hinblick auf $R^1$ und $R^2$, $R^1$ $-C_{12}H_{25}$, $-C_{14}H_{29}$, $-C_{16}H_{33}$, $-C_{18}H_{37}$ oder $-C_{20}H_{41}$ darstellt und $R^2$ ein Wasserstoffatom darstellt; oder $R^1$ ein Wasserstoffatom darstellt und $R^2$ - $(C{=}O)C_{11}H_{23}$, $-(C{=}O)C_{13}H_{27}$, $-(C{=}O)C_{15}H_{31}$, oder $-(C{=}O)C_{17}H_{35}$ darstellt, und wobei ein Gesamtgehalt an Komponente (B) und Komponente (E) 10 Masse% bis 25 Masse% beträgt.

2. Emulsionszusammensetzung nach Anspruch 1, wobei Komponente (A) Stearylglycyrrhetinat enthält.

3. Emulsionszusammensetzung nach Anspruch 1 oder 2, wobei Komponente (D) mindestens eines, ausgewählt aus der Gruppe bestehend aus einem Saccharosefettsäureester und einem Polyglycerylfettsäureester enthält.

4. Emulsionszusammensetzung nach einem der Ansprüche 1 bis 3, wobei Komponente (C) Lecithin enthält.

5. Emulsionszusammensetzung nach einem der Ansprüche 1 bis 4, wobei ein Mengenverhältnis an Komponente (C) zu einem Gesamtgehalt an Komponente (B) und Komponente (E) [C/(B+E)] die Masse betreffend 0,01 bis 0,5 beträgt.

6. Kosmetik enthaltend die Emulsionszusammensetzung nach einem der Ansprüche 1 bis 5.

7. Kosmetik nach Anspruch 6, wobei eine Absorption, die mit Licht gemessen wird, das eine Wellenlänge von 650 nm aufweist, 0,001 bis 0,2 beträgt.

## Revendications

1. Composition d'émulsion comprenant les composants suivants (A) à (E) :

(A) un dérivé d'acide glycyrrhétique représenté par la formule suivante (1) ;
(B) au moins un agent de type huile sélectionné parmi le groupe consistant en un tocophérol, un acétate de tocophérol et un tocotriénol ;
(C) un phospholipide ;
(D) un surfactant soluble dans l'eau, et
(E) un glycéride d'acide gras,

dans laquelle une teneur du dérivé d'acide glycyrrhétique représenté par la formule (1) s'étend de 1,0 % en masse à 5,0 % en masse :

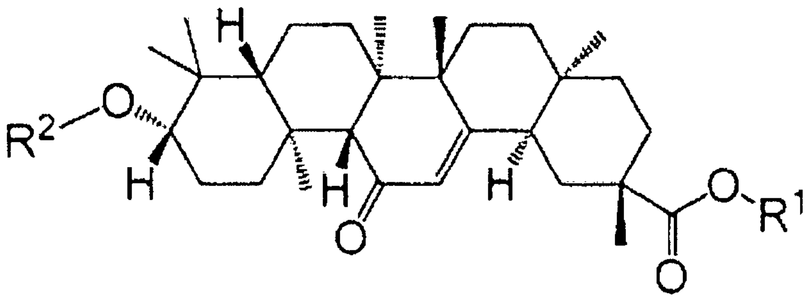

(1)

où dans la formule (1), concernant $R^1$ et $R^2$ , $R^1$ représente $-C_{12}H_{25}$, $-C_{14}H_{29}$, $-C_{16}H_{33}$, - $C_{18}H_{37}$, ou $-C_{20}H_{41}$, et $R^2$

représente un atome d'hydrogène ; ou $R^1$ représente un atome d'hydrogène et $R^2$ représente $-(C=O)C_{11}H_{23}$, $-(C=O)C_{13}H_{27}$, $-(C=O)C_{15}H_{31}$, ou $-C_{17}H_{35}$, et
dans laquelle une teneur totale du composé (B) et du composé (E) s'étend de 10 % en masse à 25 % en masse.

**2.** Composition d'émulsion selon la revendication 1, dans laquelle le composant (A) comprend du glycyrrhétinate de stéaryle.

**3.** Composition d'émulsion selon la revendication 1 ou 2, dans laquelle le composant (D) comprend au moins un élément sélectionné parmi le groupe consistant en un ester d'acide gras de saccharose et un ester d'acide gras de polyglycéryle.

**4.** Composition d'émulsion selon l'une quelconque des revendications 1 à 3, dans laquelle le composant (C) comprend une lécithine.

**5.** Composition d'émulsion selon l'une quelconque des revendications 1 à 4, dans laquelle un rapport de teneur du composant (C) à une teneur totale du composant (B) et du composant (E) [C/(B+E)] s'étend de 0,01 à 0,5 en termes de masse.

**6.** Cosmétique comprenant la composition d'émulsion selon l'une quelconque des revendications 1 à 5.

**7.** Cosmétique selon la revendication 6, dans lequel une absorbance mesurée avec une lumière présentant une longueur d'onde de 650 nm s'étend de 0,001 à 0,2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4524098 B
- JP 4341983 B
- JP 2007197328 A


### Non-patent literature cited in the description


- *CHEMICAL ABSTRACTS,* 13832-70-7